# EUROPÄISCHE PATENTANMELDUNG

(11) **EP 1 208 799 A1**
(43) Veröffentlichungstag der Anmeldung: **29.05.2002**
(21) Anmeldenummer: 00890342.9
(22) Anmeldetag: 16.11.2000
(51) Int. Cl.: A61B 8/08

(54) **Verfhren zu Bestimmung der Einführungsrichtung und zur Uberwachung des Einführungsweges von Biopsienadeln**

(71) Anmelder: Kretztechnik Aktiengesellschaft, 4871 Zipf (AT)
(72) Erfinder: Steininger, Josef, 4870 Vöcklamarkt (AT); Gritzky, Arthur, Dipl. Ing., 4710 Pollham (AT)
(74) Vertreter: Hübscher, Gerhard, Dipl.-Ing.

(57) **Zusammenfassung**

Zur Verbesserung der Einführgenauigkeit und zur leichteren Handhabung wird unter Verwendung eines 3D-Abtastkopfes und eines für die 3D-Darstellung geeigneten Ultraschallgerätes ein größerer Volumsbereich des Organismuses abgestastet und bei allfälliger Zwischenspeicherung der erhaltenen Signale in einem Volumsspeicher nach vorgebbaren Abfragekriterien aus dem Volumen die zur Anzeige gelangende Schnittebene (3a) aufgesucht, wonach in dieser Anzeige virtuell der gewünschte Einführungsweg (4) dargestellt und, insbesondere nach parallel zu der Achse des Einführungsweges verlaufenden, im Bild vorgebbaren Grenzen (5) ein den gewünschten Einführungsweg enthaltender, z.B. prismatischer oder zylindrischer Teil des Gesamtvolumens ausgewählt und in einem 3D-Bild (6) dargestellt wird, an dem auch die Biopsienadel od. dgl. bei der späteren tatsächlichen Einführung beobachtet werden kann.

## Beschreibung

Die Erfindung bezieht sich auf ein Verfahren nach dem Oberbegriff des Patentanspruches 1.

Bekannte Verfahren dieser Art arbeiten mit verhältnismäßig einfachen B-Bildgeräten, bei denen die Lage der dargestellten Schnittebene einzig und allein durch die Aufsatzstelle des Abtastkopfes und seine Ausrichtung zum untersuchten Organismus bestimmt wird. Bereits bei kleinen Verschwenkungen des Abtastkopfes um die Horizontal- oder Vertikalachse verändert sich auch naturgemäß die Lage der angezeigten Schnittebene, wobei überdies bei der Abtastung lebender Organismen durch Lageveränderungen des Gesamtkörpers, des im Körper angepeilten Organismus oder der angepeilten Körperschicht zu korrigierende Änderungen in der für die Anzeige bestimmten, den "Zielpunkt" im Organismus enthaltenden Schnittebene auftreten. Es ist bekannt, für die meist im Abstand vom Abtastkopf einzuführende, gegen die vom Abtastkopf bestimmte Schnittebene gerichtete Biopsienadel od. dgl. mit der Abtastkopf starr verbundene Führungen vorzusehen. Die gewünschte Abtastung kann im B-Bild duch eine eingeblendete Hilfslinie dargestellt werden. Auch die Biopsienadel od. dgl. selbst wird während der Einführung beobachtet. Es ist dann selbst bei ruhendem Organismus mühsam und schwierig festzustellen, ob eine nicht exakt eingeführte Biopsienadel die dargestellte Schnittebene nach vorne oder hinten verläßt und entsprechende Korrekturen vorzunehmen, wobei bei bewegten Organismen zusätzlich zur Korrektur an der Biopsienadel auch Änderungen in der Aufsetzrichtung, insbesondere -neigung des Tastkopfes, durchzuführen sind, so daß nur sehr geübte Ärzte in der Lage sind, tatsächlich die theoretisch mögliche Zielgenauigkeit bei diesen bekannten Verfahren einzuhalten. Aus der DE 24 25 724 A ist es bekannt, Biopsienadeln mit einem für Ultraschallwellen gut reflektierenden Belag auszustatten oder mit Profilierungen zu versehen, um die Sichtbarmachung im Ultraschallbild zu verbessern, doch ändert dies auch nichts an den oben angegebenen Schwierigkeiten.

Zur Erhöhung der Zielgenauigkeit wird nach der AT 344 872 B vorgesehen, zwei Abtastköpfe für eine B-Bildabtastung zu verwenden und diese etwa rechtwinkelig und mit Abstand voneinander mit einem Führungsgestänge für eine Biopsienadel zu verbinden, wobei sich die Abtastebenen der beiden Abtastköpfe im Objekt nach einer auf der durch das Führungsgestänge bestimmten Einführungsachse für die Biopsienadel liegenden Geraden schneiden. Wenn also der anzupeilende Punkt im Objekt auf der genannten Geraden liegt und in beiden Bildern angezeigt wird, so wird die eingeführte Biopsienadel mit großer Wahrscheinlichkeit diesen erwähnten Punkt treffen. Auch hier treten allerdings die schon oben erwähnten Nachstellschwierigkeiten bei bewegten oder ihre Lage verändernden Organismen auf, die Nach- und Einstellung der Abtastköpfe wird umständlich und dem behandelnden Arzt bleibt praktisch keine Hand für die Einführung der Biopsienadel frei. Außerdem ist ein derartig ausgestattetes Gerät nur für den einen Zweck verwendbar und es ist nicht immer möglich, die beiden Schallköpfe bei ausreichender bzw. guter Ankopplungsqualität am Objekt anzubringen. Zu erwähnen ist noch, daß Verfahren der gegenständlichen Art für die verschiedensten Zwecke, nämlich sowohl für die Entnahme von Flüssigkeit, Gewebe oder anderen Organismenproben, für Punktationen sowie für gezielte, im wesentlichen punktförmige Behandlungen in Organismen verwendet werden können.

Aufgabe der Erfindung ist demnach die Schaffung eines Verfahrens der eingangs genannten Art, bei dem bei weniger kritischer Aufsetzgenauigkeit des Abtastkopfes die Ziel- und Einführgenauigkeit für die Biopsienadel od. dgl. verbessert wird, eine hohe Zielgenauigkeit auch bei bewegten oder ihre Lage verändernden Organismen gewährleistet bleibt und nach dem auch weniger geübte Ärzte bei hoher Zielgenauigkeit sicher arbeiten können.

Die gestellte Aufgabe wird durch die Verfahrensmerkmale des Patentanspruches 1 vollständig gelöst. Prinzipiell kann bei der Durchführung des erfindungsgemäßen Verfahrens ein Ultraschallgerät mit entsprechendem Abtastkopf verwendet werden, wie es z. B. Gegenstand der eigenen EP 0 962 785 A ist. Dieses Gerät ist insbesondere im Softwarebereich an die Bedingungen des erfindungsgemäßen Verfahrens anzupassen, wobei zusätzlich die erwähnte Schnittebene in ihrer Gesamtheit dargestellt werden soll. Die Schnittebene kann aber vor allem bei Geräten ohne Zwangsführung für die Biopsienadel innerhalb des abgetasteten Gesamtvolumens ausgewählt werden, wobei die Einstellung nur soweit notwendig ist, daß auch das den Einführungsweg umgebende Teilvolumen sicher im abgetasteten Gesamtvolumen vorhanden bleibt. Bei Ultraschallgeräten mit Zwangsführung der Biopsienadel wird man grundsätzlich jene Schnittebene für die Darstellung auswählen, in der auch die Zielrichtung der Biopsienadelführung liegt.

Da in der 3D-Darstellung nur die tatsächlich für die Einführung der Biopsienadel bestimmten Bereiche und ihr unmittelbares Umfeld veranschaulicht werden, ist die Darstellung im Gegensatz zu einer Gesamtdarstellung weniger verwirrend, bietet aber die Möglichkeit, auch quer zur B-Bildebene Formverläufe im Organismus sichtbar zu machen, die sich z. B. aus der Oberflächenform oder aus der Form von Trennschichten ergeben, so daß der Eindringungspunkt der Biopsienadel sehr genau angepeilt werden kann. Die Nadel selbst ist infolge der 3D-Darstellung auch dann gut sichtbar, wenn sie etwas aus der Schnittbildebene herauswandert, was Korrekturen in der Nadelführung in bisher unbekannter Weise erleichtert. Bei unbewegten Organismen kann man die Begrenzungen des ausgewählten Teilvolumens verhältnismäßig eng an der Richtungsachse festlegen. Bei bewegten oder Lageveränderungen unterliegenden Organismen werden die Grenzen so abgesteckt, daß auch bei der Bewegung und ohne wesentliche Lageveränderung des Tastkopfes die Richtungsachse immer innerhalb dieses ausgewählten Teilvolumens verläuft. Daher ist eine mit dem Tatstkopf verbundene Zwangsführung für die Biopsienadel in den meisten Fällen sogar entbehrlich.

Wie schon erwähnt wurde, kann man die Grenzflächen des Teilvolumens nach verschiedenen Kriterien auswählen. Eine besonders einfache Möglichkeit ist im Unteranspruch 2 angegeben.

Die 3D-Bilddarstellung bietet verschiedene weitere Möglichkeiten zur Erhöhung der Einführgenauigkeit und auch zur genauen Bestimmung der Einführrichtung. So kann man mit an sich bekannten Maßnahmen das im 3D-Verfahren dargestellte Teilbild drehen, kippen und wenden, also von allen Seiten beobachten und auch danach schließlich die gewünschte Einstichrichtung und den Einstichpunkt wählen. Aufgrund der Gewohnheiten bei einer Bildbeobachtung kann es sich auch als vorteilhaft erweisen, wenn das 3D-Bild gemäß dem Unteranspruch 3 ausgerichtet wird.

Das erfindungsgemäße Verfahren ist nicht auf die reine Schnitt- und 3D-Bilddtechnik beschränkt, sondern kann auch bei kompatiblen Verfahren eingesetzt werden, wie dies im Anspruch 4 angegeben wurde. Bei einer Dopplercodierten Abtastung ergibt sich allerdings eine wesentliche Verringerung in den möglichen Abtastgeschwindigkeiten um die Dopplerauswertung zuzulassen.

Weitere Einzelheiten und Vorteile des Erfindungsgegenstandes entnimmt man der nachfolgenden Zeichnungsbeschreibung.

In der Zeichnung ist der Erfindungsgegenstand beispielsweise veranschaulicht. Es zeigen
- Fig. 1: schematisch die Anordnung eines 3D-Abtastkopfes an einem Objekt, wobei die abgetastete Schnittebene strichliert angedeutet wurde und die
- Fig. 2: ein nach beim erfindungsgemäßen Verfahren aus dem Volumen erzeugtes B-Bild und daneben die 3D-Darstellung des ausgewählten Teilvolumens im benachbarten Bildschirmbereich.

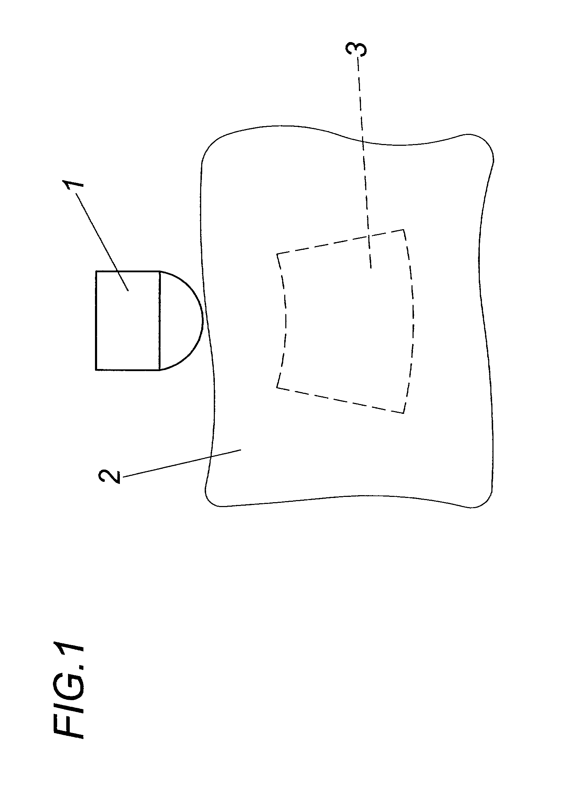

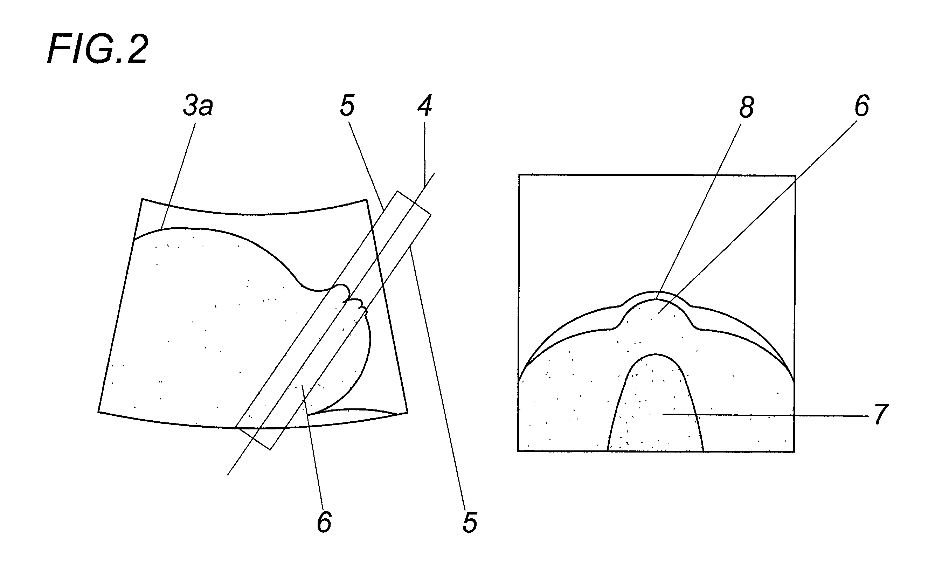

Nach Fig. 1 ist ein 3D-Tastkopf eines nicht weiter beschriebenen, dem Prinzip nach dem 3D-Bild-Gerät nach der EP 0 962 785 A entsprechenden Ultraschallgerätes auf einen Organismus 2 aufgesetzt, den es nach dem 3D-Verfahren in einem durch Verstellung einer B-Bildabtastung 3 quer zur Abtastebene erhaltenen Volumsbereich abtastet. Durch entsprechende Ableitung werden einzelne B-Bilder ausgesucht, wobei nach der bevorzugten Möglichkeit durch Einstellung des Abtastkopfes die auf dem halben Verstellweg der 3D-Abtastung quer zur Bildebene liegende Bildebene auf den Einführungsbereich einer Biopsienadel eingestellt wird, was bei vorhandener Zwangsführung für die Biopsienadel am Abtabstkopf auch notwendig ist. Sonst kann eines der weiteren B-Bilder, das den Einführungsbereich am besten darstellt, ausgewählt werden. In diesen Einführungsbereich wird nun in das B-Bild 3a nach Fig. 2 eine Hilfslinie 4 eingeblendet, die den gewünschten bzw. durch die Führung vorgegebenen Verlauf der Einführungsrichtung in die Biopsienadel anzeigt. Beidseits dieser Hilfslinie 4 werden im B-Bild ebenfalls durch Hilfslinien 5 Grenzen für ein für die 3D-Darstellung gewünschtes Teilvolumen eingezeichnet. Die Tiefe dieses Volumens kann durch den Verstellweg senkrecht zur B-Bildebene definiert werden. Ferner wird für das nun neben dem Schnittbild 3a darzustellende 3D-Bild 6 des Teilvolumens festgelegt, in welcher Richtung es zur horizontal oder vertikal darzustellenden Einführungsrichtung der Biopsienadel liegen soll und ob die 3D-Ansicht von vorne oder hinten betrachtet werden soll, wobei die linke und rechte Seitengrenze durch die Endpunkte des Verstellweges senkrecht zur B-Bildebene gegeben sind. Das Bild 6 nach Fig. 2 zeigt, daß ein Einschluß 7 im Organ und eine Erhebung 8, in die der Einstich der Biopsienadel erfolgen sollte, unvergleichlich besser und genauer als aus dem B-Bild ersichtlich ist. Die hier einzuführende Biopsienadel ist im Bild 6 ebenfalls sichtbar, wurde aber nicht dargestellt. Die Einführungsrichtung verläuft vertikal von oben nach unten in Bildmitte.

## Patentansprüche

1. Verfahren zur Bestimmung der Einführungsrichtung (4) und zur Überwachung des Einführungsweges von Biopsienadeln, Kanülen, Sonden u. dgl. in Organismen (2), unter Verwendung eines Ultraschallgerätes, wobei eine Abtasteinheit (1) des Ultraschallgerätes auf den vorgesehenen Einführungsbereich des Organismuses (2) aufgesetzt, am Gerät eine Bilddarstellung (3a) dieses Bereiches nach einer Schnittebene (3) erzeugt wird, in oder entlang der die Richtungsachse (4) des gewählten Einführungsweges verläuft und die Biopsienadel od. dgl. während ihrer tatsächlichen Einführung auf der bzw. einer Bilddarstellung (3a, 6) beobachtet wird, **dadurch gekennzeichnet, daß** unter Verwendung eines 3D-Abtastkopfes (1) und eines für die 3D-Darstellung geeigneten Ultraschallgerätes ein größerer Volumsbereich (3) des Organismuses (2) abgetastet und bei allfälliger Zwischenspeicherung der erhaltenen Signale in einem Volumsspeicher nach vorgebbaren Abfragekriterien aus dem Volumen (3) die zur Anzeige gelangende Schnittebene (3a) aufgesucht wird, wonach in dieser Anzeige virtuell der gewünschte Einführungsweg (4) dargestellt und, insbesondere nach parallel zu der Achse des Einführungsweges verlaufenden, im Bild (3a) vorgebbaren Grenzen (5) ein den gewünschten Einführungsweg enthaltender, z. B. prismatischer oder zylindrischer Teil des Gesamtvolumens ausgewählt und in einem 3D-Bild (6) dargestellt wird, an dem auch die Biopsienadel od. dgl. bei der späteren tatsächlichen Einführung beobachtet werden kann.

2. Verfahren nach Anspruch 1, **dadurch gekennzeichnet, daß** die Seitengrenzen (5) des Teilvolumens in einem B-Bild (3a) angezeigt und die vor und hinter der das B-Bild ergebenden Schnittebene (3) verlaufenden Volumsgrenzen durch die 3D-Verstellung der Abtasteinheit (1) quer zur B-Bildabtastung bestimmt werden.

3. Verfahren nach Anspruch 1 oder 2, **dadurch gekennzeichnet, daß** das 3D-Bild (6) in der Darstellung auf eine im Bild vertikal oder horizontal verlaufende Einführungsrichtung der Biopsienadel ausgerichtet wird.

4. Verfahren nach einem der Ansprüche 1 bis 3, **dadurch gekennzeichnet, daß** bei der Anzeige von nach dem Doppler-Verfahren zusätzlich codierten Schnitt- oder 3D-Bildern die Biopsienadel od. dgl. zur besseren Sichtbarmachung in dieser Art der Bilddarstellung während des Einführens bzw. Verbleibens im Organismus in Vibration versetzt wird.
